(19) Europäisches Patentamt
European Patent Office
Office européen des brevets

(11) Publication number: **0 080 682**
**A1**

(12) # EUROPEAN PATENT APPLICATION

(21) Application number: 82110782.8

(22) Date of filing: 22.11.82

(51) Int. Cl.³: C 07 D 217/20
A 61 K 31/47

(30) Priority: 23.11.81 US 324001

(43) Date of publication of application: 08.06.83 Bulletin 83/23

(84) Designated Contracting States: CH DE FR GB LI

(71) Applicant: THE WELLCOME FOUNDATION LIMITED
183-193 Euston Road
London NW1 2BP(GB)

(72) Inventor: Swaringen, Roy Archibald, Jr.
824 Sandlewood Drive
Durham North Carolina 27712(US)

(72) Inventor: El-Sayad, Hassan Ali
165 Windsor Circle
Durham North Carolina 27514(US)

(72) Inventor: Yeowell, David Arthur
608 Concordia Court
Chapel Hill North Carolina 27514(US)

(74) Representative: Berg, Wilhelm, Dr. et al,
Dr. Berg, Dipl.-Ing. Stapf, Dipl.-Ing. Schwabe, Dr. Dr.
Sandmair Mauerkircherstrasse 45
D-8000 München 80(DE)

(54) **Bisisoquinolinium compound, pharmaceutical compositions and synthesis.**

(57) Intermediate duration muscle relaxants of formula (I):

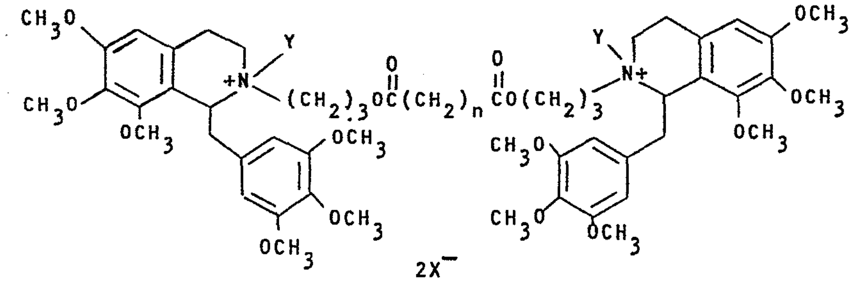

(I)

wherein Y is $C_{1-4}$ alkyl; n is 3-7; and $X^-$ is an anion; their preparation, formulations thereof and their use in producing muscle relaxation in mammals including man.

EP 0 080 682 A1

Croydon Printing Company Ltd.

TITLE MODIFIED see front page



0080682

QUARTERNARY AMMONIUM COMPOUND, PHARMACEUTICAL COMPOSITIONS AND SYNTHESIS

The present invention relates to isoquinoline derivatives, their preparation, formulations thereof and their use in medicine. In particular, this invention relates to novel isoquinoline derivatives for use as neuromuscular blocking agents.

In anaesthesia, neuromuscular blocking agents are used to provide skeletal muscle relaxation during surgery and during intubation of the trachea; hence, they are sometimes known as 'muscle relaxants'.

In general, there are two types of neuromuscular blocking agents in use: non-depolarising and depolarising.

The non-depolarising agents include d-tubocurarine, pancuronium, gallamine, diallyltoxiferine and toxiferine. The depolarising agents include succinylcholine and decamethonium.

All of the conventional non-depolarising agents when used for producing skeletal muscle relaxation in surgery have a long duration of action, e.g., 60 to 180 minutes in man. The conventional depolarising agents, on the other hand, provide muscle relaxation with a duration of action shorter than that of the non-depolarising agents. For example, succinylcholine provides a short duration of action of about 5 to 15 minutes whereas decamethonium provides about 20 to 40 minutes duration of muscle relaxation in man.

Each non-depolarising agent has inherent side effects. For example, gallamine and pancuronium may cause

tachycardia, and d-tubocurarine and diallyltoxiferine may cause hypotension.

While these drugs can be pharmacologically antagonised with anticholinesterase agents, this obviously necessitates the administration of a second drug which itself may have its own side effects, e.g., bradycardia, gut spasm and bronchorrhea. Thus, to overcome the aforementioned side effects of the anticholinesterase agents, a third drug, an anticholinergic drug, e.g., atropine, must also be given.

Therefore, attempts have been made to find new compounds which act as non-depolarising neuromuscular blocking agents and which have different durations of action, but which do not exhibit the side effects of such agents currently in use.

Thus, short-acting neuromuscular blocking agents are disclosed in Belgian patent specification No. 869 415; intermediate-duration neuromuscular blocking agents are disclosed in European patent specification No. 10-119; and both short- and intermediate-duration neuromuscular blocking agents are disclosed in British patent specification No. 2061929. Each of these compounds are isoquinoline derivatives of general formula (A):

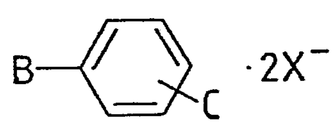

(A)

where B and C are each substituted isoquinolines.

We have now surprisingly found that potent

neuromuscular blocking agents with an intermediate-duration of action which is reversible using conventional anticholinesterase agents such as neostigmine and edrophonium and which do not exhibit the side effects of currently-used agents can be prepared which do not have the phenyl linking-group of the compounds of formula (A).

Accordingly, this invention provides a compound of formula (I):

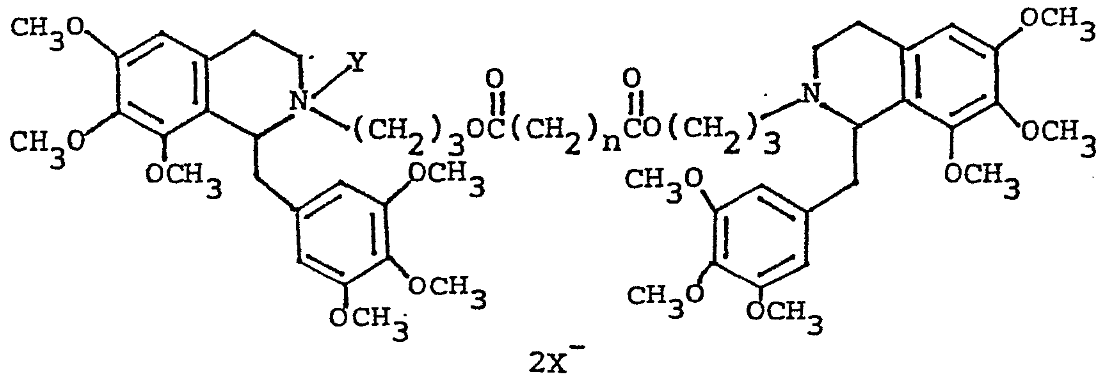

(I)

wherein Y is lower alkyl of 1 to 4 carbon atoms; n is 3 to 7; and $X^-$ is an anion.

Preferably, the trimethoxybenzyl group at the 1 position and the $(CH_2)_3$ (also known as substituted propyl) of the group $(CH_2)_3OCO(CH_2)_nOCO(CH_2)_3$ at the 2 position are in a trans relationship relative to each other in each of the nitrogen-containing rings.

Y is therefore selected from methyl, ethyl, propyl and butyl. The preferred compounds are those wherein Y is methyl.

Preferably, n is 3.

$X^-$ is preferably a pharmaceutically acceptable anion. Since the activity of the compounds of the invention resides in the di-cation, the nature of the anion $X^-$ is relatively unimportant. Suitable pharmaceutically acceptable anions include iodide, mesylate, tosylate, bromide, chloride, sulphate, phosphate, hydrogen phosphate, acetate, benzene-sulphonate, succinate, maleate, naphthalenesulphonate and propionate. Preferred anions are chloride and tosylate; thus compounds of formula (I) in the form of the dichloride, ditosylate and chloride tosylate are favoured.

The compounds of this invention are preferably provided as an approximately 1:1 mixture of the racemic (dl) pair and the meso-isomer. It is preferred that the compounds of the invention be provided in a form where the ratio of the trans, trans compound of the invention to the total of any corresponding cis, cis and cis, trans compounds present as impurities is at least 96:4.

In another aspect, the present invention provides a compound of formula (I) for use as a neuromuscular blocking agent; for example, in conjunction with surgery or for intubation of the trachea by conventional parenteral administration, e.g., intramuscular or intravenous administration in solution.

The compounds of formula (I) may be administered to mammals such as monkeys, man (humans) and other mammals to achieve a neuromuscular block. The dosage for each type of mammal will vary because of the peculiarities of the species. However, a suitable intravenous amount or dose of the compounds of formula (I) to obtain paralysis

in mammals is 0.008 to 0.2 mg/kg of body weight, preferably 0.03 to 0.1 mg/kg of body weight, the above doses being based on the weight of the di-cation which is the active ingredient. The compounds of this invention are, therefore, at least as potent as or more potent than the agents most widely used clinically (pancuronium 0.06-0.08 mg/kg, d-tubocurarine 0.4-0.6 mg/kg). The dose for intramuscular administration is two to four times the intravenous dose.

Preferably, the compound of formula (I) is administered in the form of a pharmaceutical formulation. Therefore, in a further aspect, the present invention provides a pharmaceutical formulation which comprises a compound of formula (I) in association with a pharmaceutically acceptable carrier therefor.

The compounds may be presented in a pharmaceutical formulation for parenteral administration. The formulation may be an aqueous or non-aqueous solution or emulsion in a pharmaceutically acceptable liquid or mixture of liquids, which may contain an excipient selected from bacteristatic agents, antioxidants, buffers, thickening agents, suspending agents or other pharmaceutically acceptable additives. Such formulations are normally presented in unit dose forms such as ampoules or disposable injection devices, or in multidose forms such as a bottle from which the approriate dose may be withdrawn; all such formulations should be sterile.

The compounds of this invention may be presented as a powder, e.g., as a unit dose in a sealed ampoule or vial to which sterile water or another pharmaceutically acceptable sterile liquid vehicle may be added, preferably by aseptic techniques.

A suitable unit dose to obtain a neuromuscular block for adult humans is about 2 to 15 mg and most preferably 5 to 10 mg. Thus, a suitable pharmaceutical parenteral preparation for administration to humans will preferably contain 2 to 15 mg of a compound of formula (I) in solution or multiples thereof for multidose vials.

A simple and preferred formulation is a solution of a compound of formula (I) in water which may be prepared by simply dissolving the compound in pyrogen-free water, with or without a preservative, and sterilising the solution; or by dissolving the sterile compound in pyrogen-free, sterile water under aseptic conditions.

The compounds of formula (I) may also be administered as an infusion of a dextrose solution or a saline solution, e.g., Ringer's solution. The compounds may also be administered in other solvents such as alcohol, polyethylene glycol and dimethylsulphoxide.

The compounds of formula (I) may also be administered intramuscularly as a suspension or solution.

Therefore, in another aspect, the present invention provides a method for producing a neuromuscular block in a mammal which method comprises the administration to said mammal of an effective, non-toxic, neuromuscular blocking amount of a compound of formula (I) or a pharmaceutical formulation thereof.

A preferred method of producing such a blockade in mammals is by intravenously injecting a dose of 0.008 to 0.2 mg/kg in the mammal.

The compounds of this invention if desired may be administered in conjunction with a depolarising agent

such as those listed above.

In a still further aspect, the present invention provides a method for the preparation of a compound of formula (I) which method comprises coupling an N-alkyl-N-3-hydroxypropyl-1,2,3,4,-tetrahydro-6,7,8-trimethoxy-1-(3,4,5-trimethoxybenzyl)isoquinolium salt with an appropriate dicarboxylic acid or a reactive derivative thereof such as the dicarboxylic acid anhydride or diacid chloride.

As stated above, preferably the compound of formula (I) is prepared substantially as the *trans*, *trans*-isomer, in which case the above intermediate is preferably substantially *trans*-N-alkyl-N-3-hydroxypropyl-1,2,3,4,-tetrahydro-6,7,8-trimethoxy-1-(3,4,5-trimethoxybenzyl)isoquinolium salt.

Also as stated above, preferably the compound of formula (I) is prepared as an approximately 1:1 mixture of the racemic (dl) pair and the *meso*-isomer. However, also included in this invention is the preparation of the compounds of formula (I) when in the form of one of the aforesaid isomers substantially free of the other isomers, and mixtures of one of the isomers with one or both of the other isomers. Other methods of preparing the *cis*-*trans* mixtures are well known in the art.

The present invention will now be illustrated by the following Examples in which all temperatures are in degrees Centrigrade.

Description 1

Mescaline and 3,4,5-trimethoxyphenylacetic acid were reacted in xylene to give the corresponding amide which was cyclised to the corresponding dihydroisoquinoline via

the Bischler-Napieralski reaction followed by reduction and reductive methylation to give 5',8-dimethoxylaudanosine mp 174-176°.

Description 2

5',8-dimethylaudanosine (prepared as in Description 1) (27.2 g) and 3-iodopropanol (27.2 g) were refluxed in dry acetone (150 ml) for 21 hours. The solvent was evaporated under vacuum and the unreacted 3-iodopropanol was extracted with diethyl ether (100 ml). The ether was decanted and the residue was dissolved in hot ethyl alcohol (300 ml) and cooled at 5° for 16 hours to yield 29.2 g of 9:1 mixture of the trans:cis quaternary iodides as indicated by high performance liquid chromatography (HPLC). The mixture was recrystallised twice from ethyl alcohol to give 24.4 g of trans-N-3-hydroxypropyl-5',8-dimethoxylaudanosinium iodide (98% trans by HPLC). The iodide salt was converted to the chloride salt by passing its methanolic solution through a column packed with 75 g of Dowex 1-X8 (Trade Name) ion exchange resin. The solvent was evaporated under vacuum and acetone (100 ml) was added to give 18.1 g of trans-N-3-hydroxypropyl-5',8-dimethoxylaudanosinium chloride (100% trans by HPLC). The yield was 67% overall.

Calculated for $C_{26}H_{38}NO_7Cl.2H_2O$: C, 56.98; H, 7.72; N, 2.56; Cl, 6.47. Found: C, 56.97; H, 7.74; N, 2.52; Cl, 6.47.

Example 1

Trans-N-3-hydroxypropyl-5',8-dimethoxylaudanosinium chloride (prepared as in Description 2) (>99% trans by HPLC, 2 g) was suspended in methylene chloride (50 ml) and glutarylchloride (0.31 g) was added. The mixture was heated at reflux for 210 minutes. The solvent was removed under vacuum to give an amorphous solid which was

dissolved in chloroform (100 ml) and washed with 5% aqueous sodium chloride solution (5 x 40 ml) to remove unreacted quaternary salt. The chloroform layer was dried and evaporated under vacuum. The residual amorphous solid was dissolved in water and lyophilised to give 0.99 g of *trans*, *trans*-2,2'-(trimethylenebis(carbonyloxytrimethylene))bis(1,2,3,4-tetrahydro-6,7,8-trimethoxy-2-methyl-1-(3,4,5-trimethoxybenzyl)isoquinolinium) dichloride which was assayed by high performance liquid chromatography (HPLC) as 98.4% *trans* diester, and 1.6% *cis*-*trans* diester.

Calculated for $C_{57}H_{80}N_2O_{16}Cl_2 4H_2O$: C, 57.42; H, 7.44; N, 2.35; Cl, 5.95. Found: C, 57.44; H, 7.45; N, 2.35; Cl, 5.92.

## Example 2

*Trans*-*N*-3-hydroxypropyl-5',8-dimethoxylaudanosinium chloride (>99% *trans* by HPLC, 2 g), suberic acid (0.3 g) and p-toluenesulphonic acid monohydrate (1.05 g) were refluxed in 1,2-dichloroethane (100 ml) for 42 hours while water was azeotropically removed. The solvent was removed under vacuum to give an amorphous solid which was dissolved in chloroform (50 ml) and washed with 5% aqueous sodium chloride solution (10 x 100 ml) to remove unreacted quaternary salt. The chloroform layer was evaporated under vacuum and the halfester impurity was substantially removed by trituration with acetone. Residual solvent was removed under vacuum and the resulting amorphous solid was dissolved in water and lyophilised to give 0.5 g of *trans*, *trans*-2,2'-(hexamethylenebis(carbonyloxytrimethylene))bis-(1,2,3,4-tetrahydro-6,7,8-trimethoxy-2-methyl-1-(3,4,5-trimethoxybenzyl)isoquinolinium) chloride tosylate which was assayed by high performance liquid chromatography (HPLC) as 96.9% *trans* diester, and 3.1% halfester.

JDM/ved/W821006/B359F

Calculated for $C_{60}H_{86}N_2O_{16}$ 1/4 $C_7H_7O_3S$ 7/4 Cl 4.5 $H_2O$: C, 58.07; H, 7.64; N, 2.19; S, 0.63; Cl, 4.86. Found: C, 57.99; H, 7.70; N, 2.09; S, 0.41; Cl, 4.56.

Example 3

Trans-N-3-hydroxypropyl-5',8-dimethoxylaudanosinium chloride (>97% trans by HPLC, 2 g), azelaic acid (0.32 g) and p-toluenesulphonic acid monohydrate (1.07 g) were refluxed in 1,2-dichloroethane (100 ml) for 44 hours while water was azeotropically removed. The solvent was removed under vacuum to give an amorphous solid which was dissolved in chloroform (100 ml) and washed with 1% aqueous sodium chloride solution (5 x 50 ml) to remove unreacted quaternary salt. The chloroform layer was evaporated under vacuum and the halfester impurity was substantially removed by trituration with methyl ethyl ketone. Residual solvent was removed under vacuum and the resulting amorphous solid was dissolved in methanol and lyophilised to give 1.8 g of trans, trans-2,2'-(heptamethylenebis(carbonyloxytrimethylene))bis(1,2,3,4-tetrahydro-6,7,8-trimethoxy-2-methyl-1-(3,4,5-trimethoxybenzyl)isoquinolinium) tosylate which was assayed by high performance liquid chromatography (HPLC) as 87.9% trans diester, 2.7% cis-trans diester and 8.4% halfester.

Calculated for $C_{61}H_{88}N_2O_{16}.2C_7H_7SO_3.4H_2O$: C, 59.27; H, 7.30; N, 1.84; S, 4.22. Found: C, 59.10; H, 7.40; N, 1.84; S, 3.83; Cl, 0.00.

Example 4

Trans-N-3-hydroxypropyl-5'8-dimethoxylaudanosinium chloride (>97% trans by HPLC, 2 g) was suspended in 1,2-dichloroethane (60 ml) at $\simeq$ 70°C. Sebacic acid chloride (0.42 g) was added and the mixture was stirred at ambient temperature for 17 hours. The solvent was removed under vacuum to give an amorphous solid which was dissolved in

chloroform (100 ml) and washed with water (2 x 50 ml) to remove unreacted quaternary salt. The chloroform layer was dried and evaporated under vacuum to give an amorphous solid. The halfester impurity was removed by washing with hot acetone. Residual solvent was evaporated under vacuum and the resulting amorphous solid was dissolved in methanol, filtered and evaporated under high vacuum to give 1.1 g of trans, trans-2,2'-(octamethylene-bis(carbonyloxytrimethylene))bis(1,2,3,4-tetrahydro-6,7,-8-trimethoxy-2-methyl-1-(3,4,5-trimethoxybenzyl)isoquinolinium) dichloride which was assayed by high performance liquid chromatography (HPLC) as 95.8% trans diester, 3.1% cis-trans diester and 4.2% halfester.

Calculated for $C_{62}H_{90}N_2O_{16}Cl_2 4H_2O$: C, 59.00; H, 7.82; N, 2.22; Cl, 5.55. Found: C, 58.98; H, 7.86; N, 2.22; Cl, 5.60.

Example 5

Trans, trans-2,2'-(trimethylenebis(carbonyloxytrimethylene))bis(1,2,3,4-tetrahydro-6,7,8-trimethoxy-2-methyl-1-(3,4,5-trimethoxybenzyl)isoquinolinium) dichloride (prepared as in Example 3) was examined by intravenous administration to cats and Rhesus monkeys, maintained by artificial ventilation and prepared for recording the isometric twitch of the tibialis anterior muscle in response to stimulation of peroneal nerve. The results are shown in the following table.

| Cat | | | Rhesus Monkey | | |
|---|---|---|---|---|---|
| No. of Animals | $ED_{95}$ (mg/kg) | *Duration (Minutes) | No.of Animals | $ED_{95}$ (mg/kg) | *Duration (Minutes) |
| 2 | 0.03 | $\simeq$25 | 4 | 0.05 | $\simeq$15$^{+}$ |

* The time from injection to 95% recovery.
+ This translates to about 50 minutes in man.

Claims:

1. A compound of formula (I):

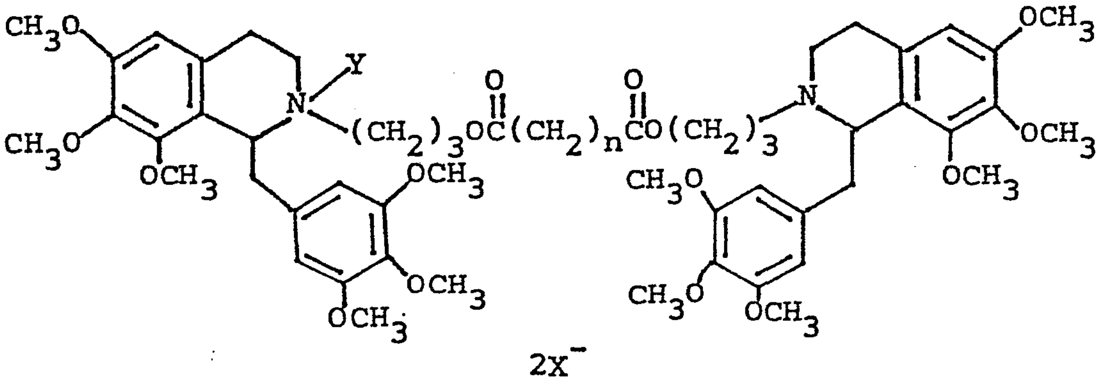

(I)

wherein Y is lower alkyl of 1 to 4 carbon atoms, n is 3 to 7 and X is an anion.

2. A compound according to claim 1 wherein the trimethoxybenzyl group at the 1 position and the substituted propyl of the group $(CH_2)_3OCO(CH_2)_nOCO(CH_2)_3$ at the 2-position are in a <u>trans</u> relationship to each other in each of the nitrogen-containing rings.

3. A compound mixture comprising the mixture of the racemic (dl) pair and the meso-isomer of a compound of formula (I) as defined in claim 1.

4. A compound according to any of claims 1 to 3 wherein Y is methyl.

5. A compound according to any of claims 1 to 4 wherein n is 3.

6. A compound selected from: _trans_, _trans_-2,2'-(trimethylenebis(carbonyloxytrimethylene))bis-(1,2,3,4-tetrahydro-6,7,8-trimethoxy-2-methyl-1-(3,4,5-trimethoxybenzyl)isoquinolinium) dichloride;
_trans_, _trans_-2,2'-(hexamethylenebis (carbonyloxytrimethylene))bis(1,2,3,4-tetrahydro-6,7,8-trimethoxy-2-methyl-1-(3,4,5-trimethoxybenzyl) isoquinolinium) chloride tosylate;
_trans_, _trans_-2,2'-(heptamethylenebis(carbonyloxytrimethylene))bis(1,2,3,4-tetrahydro-6,7,8-trimethoxy-2-methyl-1-(3,4,5-trimethoxybenzyl)isoquinolinium) tosylate; and
_trans_, _trans_-2,2'-(octamethylenebis(carbonyloxytrimethylene))bis(1,2,3,4-tetrahydr-o-6,7,8,trimethoxy-2-methyl-1-(3,4,5-trimethoxybenzyl)isoquinolinium) dichloride.

7. A method for the preparation of a compound according to any of claims 1 to 6 which method comprises coupling an _N_-alkyl-_N_-3-hydroxypropyl-1,2,3,4-tetrahydro-6,7,8-trimethoxy-1-(3,4,5-trimethoxybenzyl)isoquinolinium salt with an appropriate dicarboxylic acid or a reactive derivative thereof.

8. A pharmaceutical formulation which comprises a compound according to any of claims 1 to 6 in association with a pharmaceutically acceptable carrier therefor.

9. A formulation according to claim 8 in a form suitable for intravenous or intramuscular administration.

10. A compound according to any of claims 1 to 6 for

use as a neuromuscular blocking agent.

European Patent Office

# EUROPEAN SEARCH REPORT

0080682
Application number

EP 82 11 0782

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | US-A-3 004 031 (ALLEN & HANBURYS) *Claim 1; column 4, lines 70-75; column 5; column 6, lines 1-15* | 1,8 | C 07 D 217/20<br>A 61 K 31/47 |
| Y,D | GB-A-2 002 758 (MASSACHUSETTS GENERAL HOSPITAL) *Claim 1; page 2, lines 27-44; page 3, lines 1-8; example 6* | 1,8 | |
| P,A | EP-A-0 054 309 (WELLCOME) *Claims 1,8* | 1,8 | |

TECHNICAL FIELDS SEARCHED (Int. Cl. 3)

C 07 D 217/00
A 61 K 31/00

The present search report has been drawn up for all claims

| Place of search | Date of completion of the search | Examiner |
|---|---|---|
| THE HAGUE | 24-02-1983 | ALFARO I. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82